# EUROPÄISCHE PATENTSCHRIFT

(11) **EP 1 315 461 B1**
(45) Veröffentlichungstag und Bekanntmachung des Hinweises auf die Patenterteilung: **08.08.2007**
(21) Anmeldenummer: 01965249.4
(22) Anmeldetag: 05.09.2001
(51) Int. Cl.: A61B 17/34

(54) **VORRICHTUNG ZUR ABDICHTUNG UND SCHMIERUNG VON IN TROKARHÜLSEN GEFÜHRTEN INSTRUMENTEN**
DEVICE FOR THE SEALING AND LUBRICATION OF INSTRUMENTS RUNNING IN TROCAR SLEEVES
DISPOSITIF POUR ETANCHEIFIER ET LUBRIFIER DES INSTRUMENTS GUIDES DANS DES GAINES DE TROCART

(30) Priorität: 06.09.2000 DE 20015388 U
(43) Veröffentlichungstag der Anmeldung: 04.06.2003
(73) Patentinhaber: Karl Storz GmbH & Co. KG, 78532 Tuttlingen (DE)
(72) Erfinder: STREIFINGER, Wolfgang, 86316 Friedberg-Stätzling (DE); STORM, Gerald, 86153 Augsburg (DE)
(74) Vertreter: Riebling, Peter
(86) Internationale Anmeldenummer: PCT/EP2001/010190
(87) Internationale Veröffentlichungsnummer: WO 2002/019929

(56) Entgegenhaltungen:
- US-A- 3 871 358
- US-A- 5 334 166
- US-A- 5 535 745
- US-A- 5 720 730
- US-A- 5 941 815

## Beschreibung

Die Erfindung betrifft eine Vorrichtung zur Abdichtung und Schmierung von in Trokaren oder Trokarhülsen geführten Instrumenten nach dem Oberbegriff des unabhängigen Patentanspruchs 1.

Bei minimalinvasiven Operationen, z.B. Operationen in Bauchspiegeltechnik, werden Trokarhülsen in die Bauchdecke eingebracht, um durch diese Zugänge eine Videooptik und z.B. chirurgische Instrumente in die Bauchhöhle einführen zu können. Die Trokarhülsen schließen gegen die Bauchdecke und mittels einer Abschlusskappe gegen die eingeführten Instrumente gasdicht ab, so dass die Bauchhöhle durch insuffliertes CO₂-Gas aufgeblasen werden kann. In dem so entstandenen Raum kann mit den durch die Trokarhülse eingeführten Instrumenten unter Videokontrolle operiert werden.

Die eingeführten Instrumente gleiten ständig durch die Bewegungen beim Präparieren an der äußeren Dichtlippe der Abschlusskappe der Trokarhülse , entlang. Die Dichtlippe wird normalerweise mit einem Tropfen Paraffinöl benetzt, damit das Instrument gut gleitet und die Dichtlippe gasdicht am Instrument abschließt. Wird diese Dichtzone trocken, so erhöht sich die Reibung, was das Präparieren erschwert. Während der Operation muss also ständig Öl nachgetropft werden, um die oben genannten nachteiligen Auswirkungen zu vermeiden. Dieser momentane Zustand fordert ein gewisses Grundmaß an Aufmerksamkeit und Arbeitseinsatz; dies gilt es zu eliminieren.

Die US 3 871 358 A offenbart eine Führungshülse in dessen Einlassbereich ein mit einem Schmiermittel getränkter zylindrischer Schaumstoffkörper angeordnet ist.

Aus der US 5 941 815 A ist eine Führungshülse für chirurgische Instrumente bekannt, an deren beiden Enden mit Schmiermittel getränkte Schaumstoffdichtungen vorgesehen sind.

Die US 5 535 745 A offenbart eine Führungshülse für ein Stilett, in der eine Art Schmierkissen angeordnet ist, das von dem Stilett durchstoßen wird.

In der US 5 334 166 A ist eine Einrichtung zum Schmieren von Führungsdrähten und Kathetern beschrieben, wobei hierzu ein mit Schmiermittel getränkter Schaumstoffkörper verwendet wird.

Bei den im Stand der Technik beschriebenen Führungs- und Schmiereinrichtungen ist oftmals die Gasdichtheit nicht gegeben. Dies soll mit der vorliegenden Erfindung verbessert werden.

Die Aufgabe der Erfindung besteht darin, eine Vorrichtung zur Abdichtung und Schmierung von in Trokaren oder Trokarhülsen geführten Instrumenten vorzuschlagen, welche gasdicht, pflegeleicht und selbstschmierend ist.

Diese Aufgabe wird durch die im Patentanspruch 1 angegebenen Merkmale gelöst.

Erfindungsgemäß ist in der Abschlusskappe mindestens eine zweite Dichtlippe angeordnet, wobei der Körper zwischen den Dichtlippen angeordnet und gehalten ist.

Die Abschlusskappe weist also einen innen einliegenden Körper aus schwamm- oder vliesartigem Material auf - nachfolgend auch allgemein als Schwämmchen bezeichnet -, der den Außenumfang des eingeführten Instruments vorzugsweise vollständig umgibt. Hierzu ist das Schwämmchen vorzugsweise ringförmig ausgebildet, im Außendurchmesser der Abschlusskappe und im Innendurchmesser den einzuführenden Instrumenten angepasst.

Das eingeführte Instrument gleitet nun mühelos, von der Reservoir-Wirkung des mit Schmiermittel getränkten Schwämmchens dauerhaft geschmiert und abgedichtet, durch die Trokarhülse.

In einer bevorzugten Ausbildung der Erfindung wird der schwammartige Körper vor Gebrauch mit dem Schmiermittel getränkt und in die Abschlusskappe eingesetzt, oder liegt schon gebrauchsfertig getränkt, z.B. abgekapselt als gebrauchsfertiger Einmalartikel, vor.

Die Abschlusskappe zusammen mit dem mit Schmiermittel getränkten Körper kann vorzugsweise als gebrauchsfertiger Einmalartikel vorliegen.

Alternativ kann die Abschlusskappe resterilisierbar und mehrfach verwendbar ausgebildet sein, wogegen das Schwämmchen jedes Mal neu eingesetzt wird.

Eine andere mögliche Ausbildung der Erfindung sieht vor, dass die Abschlusskappe einen Schmiernippel und/oder eine Schmiermittelzufuhr zum Benetzen des bereits eingesetzten schwammartigen Körpers mit dem Schmiermittel aufweist. Das Schwämmchen kann so bei langen Operationen mehrmals mit dem Schmiermittel nachgetränkt werden.

Als Schmiermittel kann jedes geeignete, bioverträgliche, inerte Schmiermittel, wie z.B. Paraffinöl, eingesetzt werden.

Der schwammartige Körper selbst besteht verständlicherweise ebenfalls aus einem chemisch und biologisch inerten Material und ist ein Einweg-Artikel, der nach bekannten Verfahren sterilisierbar sein muss. Vor Gebrauch wird das Schwämmchen aus seiner sterilen Verpackung entnommen und in die dafür vorgesehene Passung in der Abschlusskappe eingelegt.

In einer bevorzugten Ausführungsform der Erfindung ist die Abschlusskappe mehrteilig ausgebildet, wobei ein erster Teil passgenau auf die Trokarhülse aufsetzbar ist und ein zweiter, den schwammartigen Körper aufnehmende Teil mit dem ersten Teil lösbar verbunden ist. Diese mehrteilige Ausformung erleichtert die Handhabung und das Einbringen des Schwämmchens.

Gemäss dieser Ausführungsform weisen der erste und der zweite Teil der Abschlusskappe je mindestens eine Dichtlippe auf, wobei der schwammartige Körper zwischen den Dichtlippen der miteinander verbundenen Teile angeordnet und gehalten ist. Das eingeführte Instrument gleitet zwischen den Dichtlippen geschmiert durch das im Schwämmchen enthaltene Schmiermittel hindurch.

Die Erfindung kann auch zur Abdichtung und Schmierung von in Einweg-Trokaren geführten Instrumenten verwendet werden. Diese Einweg-Trokare werden nur einmal benutzt. Innerhalb der Trokarhülse kann ein weiterer mit einem Schmiermittel getränkter schwamm- oder vliesartiger Körper angeordnet sein.

Nachfolgend wird ein bevorzugtes Ausführungsbeispiel der Erfindung anhand der Zeichnungsfiguren erläutert. Aus den Zeichnungen und deren Beschreibung ergeben sich weitere Merkmale und Vorteile der Erfindung.

Es zeigt:
- Figur 1:: Eine Seitenansicht der Vorrichtung;
- Figur 2:: Einen seitlichen Schnitt durch die Vorrichtung;
- Figur 3:: Einen Schnitt durch das Schwämmchen;
- Figur 4:: Eine Draufsicht auf das Schwämmchen.

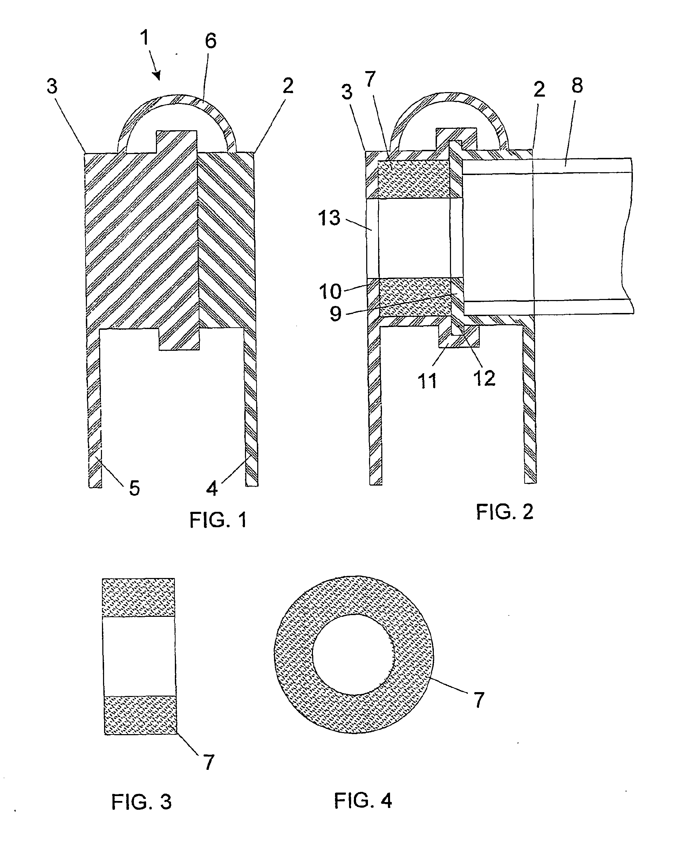

Figur 1 zeigt eine bevorzugte Ausführungsform der erfindungsgemäßen Vorrichtung. Die Abschlusskappe 1 umfasst dabei zwei Teile 2, 3, die vorzugsweise über eine Verbindungslasche 6 unverlierbar verbunden sind. Jedes der Teile 2, 3 der Abschlusskappe umfasst eine Haltelasche 4 bzw. 5 zur besseren Handhabung der Teile.

Wie in Figur 2 gezeigt ist, ist das erste Teil 2 so ausgeformt, dass es passgenau auf das Einführende der Trokarhülse 8 aufgesteckt werden kann. An einem Ende umfasst das Teil 2 eine Dichtlippe 9, durch deren Öffnung ein Operationsinstrument in die Trokarhülse 8 eingeführt werden kann. Die Dichtlippe 9 liegt dabei am Außenumfang des Operationsinstrumentes an. Mit dem Teil 2 wird ein weiteres Teil 3 verbunden. Hierzu weist das Teil 2 einen umlaufenden Bund 12 auf, der passgenau in eine Innennut des Bundes 11 des zweiten Teiles 3 eingesteckt werden kann. Somit sind die beiden Teile fest aber lösbar miteinander verbunden. Das zweite Teil 3 weist ebenfalls eine Dichtlippe 10 auf, durch deren Öffnung 13 das Operationsinstrument einführbar ist.

Zwischen der Dichtlippe 9 des Teiles 2 und der Dichtlippe 10 des Teiles 3 bildet sich ein Hohlraum, in welchem ein saugfähiges Material, vorzugsweise ein Schwämmchen 7, angeordnet ist. Wie aus den Figuren 3 und 4 ersichtlich ist, ist das Schwämmchen 7 vorzugsweise ringförmig ausgebildet. Der Innendurchmesser des Schwämmchen 7 ist dabei an den Außendurchmesser des Operationsinstrumentes angepasst. Da es sich bei dem Schwämmchen 7 um ein elastisches Material handelt, kann der Innendurchmesser des Schwämmchens 7 auch kleiner sein als der Außendurchmesser des Operationsinstrumentes, da das Schwämmchen 7 sich beim Einführen des Instrumentes in die Öffnung 13 weitet. Der Außendurchmesser des Schwämmchens 7 entspricht dem inneren Durchmesser des Teiles 3 der Abschlusskappe 1.

Das Schwämmchen 7 wird vor dem operativen Eingriff mit einem Schmiermittel, vorzugsweise Paraffinöl, getränkt und in die Passung des Teiles 3 eingelegt. Ein in die Trokarhülse 8 eingeführtes Instrument gleitet nun durch die Öffnung 13 entlang der Dichtlippe 10, entlang dem Innendurchmesser des Schwämmchens 7 und entlang der Dichtlippe 9, und wird dabei durch das Schwämmchen 7 mit Schmiermittel benetzt. Dies stellt zum einen ein reibungsfreies Gleiten des Instrumentes in der Trokarhülse 8 sicher und zum anderen eine einwandfreie Dichtigkeit zwischen dem Instrument und den Dichtlippen 9 bzw. 10.

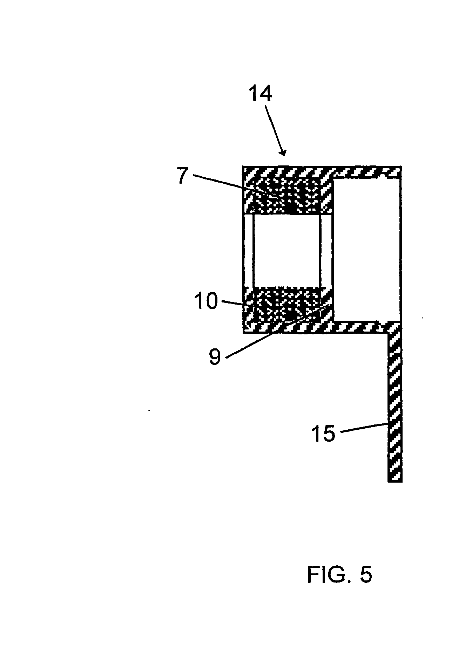

In Figur 5 ist im Gegensatz zu den Figuren 1 und 2 eine einteilige Abschlusskappe 14 dargestellt, die vorzugsweise als gebrauchsfertiger Einweg-Artikel ausgebildet ist. Die Abschlusskappe 14 weist wiederum zwei Dichtlippen 9, 10 auf, zwischen denen das bereits mit Schmiermittel getränkte Einweg-Schwämmchen 7 angeordnet ist. Zur leichteren Handhabung der Anordnung dient eine Haltelasche 15.

### Zeichnungslegende

- 1: Abschlusskappe
- 2: Teil
- 3: Teil
- 4: Haltelasche
- 5: Haltelasche
- 6: Verbindungslasche
- 7: Schwämmchen
- 8: Trokarhülse
- 9: Dichtlippe
- 10: Dichtlippe
- 11: Bund
- 12: Bund
- 13: Öffnung
- 14: Abschlusskappe
- 15: Haltelasche

## Patentansprüche

1. Vorrichtung zur Abdichtung und Schmierung von in Trokarhülsen geführten Instrumenten, mit einer auf das Einführende der Trokarhülse (8) passgenau aufsetzbaren Abschlusskappe (1), in der ein mit einem Schmiermittel benetzbarer Körper (7) aus schwamm- oder vliesartigem Material angeordnet ist, der zumindest teilweise am Außenumfang eines eingeführten Instruments anliegen kann, wobei in der Abschlusskappe (1) eine Dichtlippe (9) angeordnet ist;
**dadurch gekennzeichnet,**
**dass** in der Abschlusskappe (1) mindestens eine zweite Dichtlippe (10) angeordnet ist, und der Körper (7) zwischen den Dichtlippen (9; 10) angeordnet und gehalten ist.

2. Vorrichtung nach Anspruch 1, **dadurch gekennzeichnet, dass** die Abschlusskappe (14) ein steriler Einweg-Artikel ist.

3. Vorrichtung nach Anspruch 1, **dadurch gekennzeichnet, dass** die Abschlusskappe (1) resterilisierbar und mehrfach verwendbar ist.

4. Vorrichtung nach einem der vorhergehenden Ansprüche, **dadurch gekennzeichnet, dass** die Abschlusskappe (1; 14) einen Schmiernippel und/oder eine Schmiermittelzufuhr zum Benetzen des Körpers (7) mit dem Schmiermittel aufweist.

5. Vorrichtung nach einem der vorhergehenden Ansprüche, **dadurch gekennzeichnet, dass** die Abschlusskappe (1) mehrteilig ausgebildet ist, wobei ein erster Teil (2) passgenau auf die Trokarhülse aufsetzbar ist und ein zweiter, den schwamm oder vliesartigen Körper (7) aufnehmende Teil (3) mit dem ersten Teil (2) lösbar verbunden ist.

6. Vorrichtung nach einem der vorhergehenden Ansprüche, **dadurch gekennzeichnet, dass** der erste und der zweite Teil (2; 3) der Abschlusskappe (1) je mindestens eine Dichtlippe (9; 10) aufweisen, wobei der Körper (7) zwischen den Dichtlippen (9; 10) der miteinander verbundenen Teile (2; 3) angeordnet und gehalten ist.

7. Vorrichtung nach einem der vorhergehenden Ansprüche, **dadurch gekennzeichnet, dass** innerhalb der Trokarhülse (8) ein weiterer mit Schmiermittel getränkter schwamm- oder vliesartiger Körper angeordnet ist der zumindest teilweise am Außenumfang eines eingeführten Instruments anliegen kann.

8. Vorrichtung nach einem der vorhergehenden Ansprüche, **dadurch gekennzeichnet, dass** der Körper (7) mit dem Schmiermittel getränkt ist.

9. Vorrichtung nach einem der vorhergehenden Ansprüche, **dadurch gekennzeichnet, dass** das Schmiermittel ein bioverträgliches, inertes Schmiermittel, wie Paraffinöl, ist.

10. Vorrichtung nach einem der vorhergehenden Ansprüche, **dadurch gekennzeichnet, dass** der Körper (7) ringförmig ausgebildet ist.

11. Vorrichtung nach einem der vorhergehenden Ansprüche, **dadurch gekennzeichnet, dass** der Körper (7) aus einem chemisch und biologisch inerten Material besteht.

12. Vorrichtung nach einem der vorhergehenden Ansprüche, **dadurch gekennzeichnet, dass** der Körper (7) ein steriler Einweg-Artikel ist.

## Claims

1. Device for the sealing and lubrication of instruments guided in trocar sleeves, comprising a sealing cap (1), which can be placed to fit precisely on the insertion end of the trocar sleeve (8), in which cap a body (7) made of a sponge-like or fleece-like material, which can be wetted with a lubricating agent, is arranged and can rest at least partially on the outer periphery of an inserted instrument, a sealing lip (9) being arranged in the sealing cap (1), **characterised in that** at least a second sealing lip (10) is arranged in the sealing cap (1), and the body (7) is arranged and held between the sealing lips (9; 10).

2. Device according to claim 1, **characterised in that** the sealing cap (14) is a sterile disposable article.

3. Device according to claim 1, **characterised in that** the sealing cap (1) can be resterilised and can be used several times.

4. Device according to any one of the preceding claims, **characterised in that** the sealing cap (1; 14) has a lubricating nipple and/or a lubricating agent supply for wetting the body (7) with the lubricating agent.

5. Device according to any one of the preceding claims, **characterised in that** the sealing cap (1) is multi-part, wherein a first part (2) can be placed to fit precisely on the trocar sleeve and a second part (3) receiving the sponge-like or fleece-like body (7) is detachably connected to the first part (2).

6. Device according to any one of the preceding claims, **characterised in that** the first and the second part (2; 3) of the sealing cap (1) in each case have at least one sealing lip (9; 10), the body (7) being arranged and held between the sealing lips (9; 10) of the parts (2; 3) connected to one another.

7. Device according to any one of the preceding claims, **characterised in that** a further sponge-like or fleece-like body saturated with lubricating agent is arranged inside the trocar sleeve (8) and can rest at least partially on the outer periphery of an inserted instrument.

8. Device according to any one of the preceding claims, **characterised in that** the body (7) is saturated with the lubricating agent.

9. Device according to any one of the preceding claims, **characterised in that** the lubricating agent is a biocompatible, inert lubricating agent, such as paraffin oil.

10. Device according to any one of the preceding claims, **characterised in that** the body (7) is annular.

11. Device according to any one of the preceding claims, **characterised in that** the body (7) consists of a chemically and biologically inert material.

12. Device according to any one of the preceding claims, **characterised in that** the body (7) is a sterile disposable article.

## Revendications

1. Dispositif pour étanchéifier et lubrifier des instruments guidés dans des gaines de trocart, comportant un couvercle (1) qui est apte à être posé de manière ajustée sur l'extrémité d'introduction de la gaine de trocart (8) et dans lequel est disposé un corps (7) composé d'une matière de type éponge ou non tissé, qui est apte à être humecté avec un lubrifiant et qui peut être appliqué au moins partiellement contre la circonférence extérieure d'un instrument introduit, une lèvre d'étanchéité (9) étant disposée dans le couvercle (1),
**caractérisé en ce qu'**au moins une seconde lèvre d'étanchéité (10) est disposée dans le couvercle (1), et le corps (7) est disposé et maintenu entre les lèvres d'étanchéité (9 ; 10).

2. Dispositif selon la revendication 1, **caractérisé en ce que** le couvercle (14) est un article stérile à usage unique.

3. Dispositif selon la revendication 1, **caractérisé en ce que** le couvercle (1) est apte à être restérilisé et est utilisable plusieurs fois.

4. Dispositif selon l'une des revendications précédentes, **caractérisé en ce que** le couvercle (1 ; 14) présente un embout de lubrification et/ou une amenée de lubrifiant pour humecter le corps (7) avec le lubrifiant.

5. Dispositif selon l'une des revendications précédentes, **caractérisé en ce que** le couvercle (1) est en plusieurs parties, une première partie (2) pouvant être posée de manière ajustée sur la gaine de trocart tandis qu'une seconde partie (3) qui reçoit le corps en forme d'éponge ou de non tissé (7) est reliée de manière amovible à la première partie (2).

6. Dispositif selon l'une des revendications précédentes, **caractérisé en ce que** les première et seconde parties (2 ; 3) du couvercle (1) présentent chacune au moins une lèvre d'étanchéité (9 ; 10), le corps (7) étant disposé et maintenu entre les lèvres d'étanchéité (9 ; 10) des parties reliées (2 ; 3).

7. Dispositif selon l'une des revendications précédentes, **caractérisé en ce qu'**il est prévu à l'intérieur de la gaine de trocart (8) un autre corps en forme d'éponge ou de non tissé qui est imprégné de lubrifiant et qui peut être appliqué au moins partiellement contre la circonférence extérieure d'un instrument introduit.

8. Dispositif selon l'une des revendications précédentes, **caractérisé en ce que** le corps (7) est imprégné de lubrifiant.

9. Dispositif selon l'une des revendications précédentes, **caractérisé en ce que** le lubrifiant est un lubrifiant inerte à compatibilité biologique, tel que de l'huile de paraffine.

10. Dispositif selon l'une des revendications précédentes, **caractérisé en ce que** le corps (7) a une forme annulaire.

11. Dispositif selon l'une des revendications précédentes, **caractérisé en ce que** le corps (7) se compose d'une matière chimique et biologiquement inerte.

12. Dispositif selon l'une des revendications précédentes, **caractérisé en ce que** le corps (7) est un article stérile à usage unique.
